# EUROPEAN PATENT SPECIFICATION

(11) **EP 4 130 844 B1**
(45) Date of publication and mention of the grant of the patent: **28.01.2026**
(21) Application number: 21774948.0
(22) Date of filing: 26.03.2021
(51) Int. Cl.: G02B 23/24, A61B 1/00, A61B 17/00

(54) **ENDOSCOPE**
ENDOSKOP
ENDOSCOPE

(30) Priority: 27.03.2020 JP 2020057091
(43) Date of publication of application: 08.02.2023
(73) Proprietor: FUJIFILM Corporation, Tokyo 106-8620 (JP)
(72) Inventor: ONO, Hirotoshi, Ashigarakami-gun, Kanagawa 258-8538 (JP); SATO, Jun, Ashigarakami-gun, Kanagawa 258-8538 (JP)
(74) Representative: Dehns Germany Partnerschaft mbB
(86) International application number: PCT/JP2021/012961
(87) International publication number: WO 2021/193943

(56) References cited:
- WO-A1-2014/103829
- JP-A- 2005 027 896
- JP-A- 2010 234 058
- JP-A- 2013 150 823
- JP-A- 2015 093 052
- JP-A- 2018 143 583
- US-A1- 2014 357 947
- US-A1- 2016 227 984
- US-A1- 2019 374 288

## Description

### BACKGROUND OF THE INVENTION

### 1. Field of the Invention

The present invention relates to an endoscope.

### 2. Description of the Related Art

An endoscope is widely used in the medical and industrial fields. An endoscope used in the medical field generally comprises an insertion part to be inserted into a subject and an operation part consecutively provided at a proximal end portion of the insertion part. The insertion part has a bendable portion that is bendable, and an operation member provided on the operation part is operated so that the bendable portion is made bendable.

An operator who operates the endoscope grips the operation part, and inserts the insertion part into the subject to perform observation and the like. When the operator performs the observation using the endoscope, the operator may perform the insertion or observation while changing the direction of the distal end portion of the insertion part or the bending direction thereof by bending the bendable portion and by rotating the insertion part around an axis with respect to the operation part. As shown in Fig. 15, in an endoscope disclosed in JP2010-234058A, an annular rotary knob 119 is rotatably provided at a connection portion 118 between an insertion part 112 and an operation part 116. The insertion part 112 is provided with a bendable portion 114 that is bent by the rotational movement operation of a bending lever 121. A cover protruding portion 126 is formed on the operation part 116. The insertion part 112 is rotated with respect to the operation part 116 by the rotation operation of the rotary knob 119. A knob protruding portion 135 is formed on the rotary knob 119.

In the endoscope disclosed in JP2010-234058A, it can be seen that the insertion part 112 is in a neutral position with respect to the operation part 116 in a case where an indicator (not shown) formed on the cover protruding portion 126 and an indicator (not shown) formed on the knob protruding portion 135 are aligned with each other. The cover protruding portion 126 and the knob protruding portion 135 are formed in sizes and shapes different from each other. In addition, in an endoscope disclosed in JP2004-305413A, indicators that are used to grasp the direction of an insertion part are provided in an operation part and a knob portion for performing an operation to rotate the insertion part, respectively.

Further, in an endoscope disclosed in JP1995-345A (H07-345A), a switching ring for switching the insertion part from a state in which the insertion part is rotationally movable with respect to the operation part to a state in which the insertion part is rotationally moved and biased, and a fixing ring for switching the insertion part from the state in which the insertion part is rotationally movable to a state in which the insertion part is rotationally unmovable are provided. Indicators are formed on the switching ring and the insertion part, respectively. In a case where the indicators are aligned with each other by the rotational movement operation of the switching ring, the rotational movement biasing of a built-in coil spring can act to return the insertion part to a fixed position (neutral position). Then, after the insertion part is returned to the fixed position, the position of the insertion part can be fixed by the rotational movement operation of the fixing ring.

### SUMMARY OF THE INVENTION

The operator's visual line faces a monitor or a person to be examined during the observation using the endoscope. However, the endoscopes disclosed in JP2010-234058A, JP2004-305413A, and JP1995-345A (H07-345A) have a configuration in which the insertion part is returned to the neutral position by the alignment of the positions of the indicators, and it is very difficult to perform an operation to align the positions of the indicators with each other while looking at the positions during the observation.

Further, in the endoscope disclosed in JP2010-234058A, it is difficult to determine whether or not the fingertip touches the cover protruding portion 126 only by the feel because the cover protruding portion 126 is thinner than the knob protruding portion 135 and has a small amount of protrusion. Since the cover protruding portion 126 and the knob protruding portion 135 have shapes and sizes different from each other, there is a difference in level between both the cover protruding portion 126 and the knob protruding portion 135 when the cover protruding portion 126 and the knob protruding portion 135 are returned to the neutral position. That is, since the operator feels the difference in level even in a case where the operator tries to align the positions of the cover protruding portion 126 and the knob protruding portion 135 with each other only by the feel of the fingertip, the operator eventually visually confirms the positions.

An object of the present invention is to provide an endoscope in which an operator can easily return an insertion part to a neutral position by rotationally moving the insertion part without visually confirming the endoscope.

According to an aspect of the present invention, there is provided an endoscope as claimed in claim 1.

It is preferable that a plurality of the finger rest protruding portions are provided on the insertion part-side operation member, and that one of the finger rest protruding portions is located at a position 90° apart from the insertion part-side protruding portion with the rotational movement axis as a center.

It is preferable that a second protrusion amount in which the finger rest protruding portion protrudes from an outer peripheral surface of the insertion part-side operation member in a radial direction passing through the finger rest protruding portion is smaller than a first protrusion amount in which the insertion part-side protruding portion protrudes from the outer peripheral surface of the insertion part-side operation member in a radial direction passing through the insertion part-side protruding portion. It is more preferable that a length of the finger rest protruding portion is greater than a length of the insertion part-side protruding portion, in an axial direction along the rotational movement axis.

It is preferable that the operation part is provided with a bending operation lever that is operated to move rotationally, and that the insertion part is located at a neutral position in which a plane including a rotational movement direction of the bending operation lever and a plane including a bending direction of the bendable portion are the same as or parallel to each other, in a case where the end surfaces of the operation part-side protruding portion and the insertion part-side protruding portion face each other and the contour lines coincide with each other.

It is preferable that a plurality of the finger rest protruding portions are provided on the insertion part-side operation member, and are provided at positions symmetrical to each other with respect to a center line of the insertion part-side protruding portion orthogonal to the rotational movement axis.

It is preferable that three finger rest protruding portions are provided on the insertion part-side operation member, and that the insertion part-side protruding portion and the finger rest protruding portions are disposed in an X shape centered on the rotational movement axis.

It is preferable that the insertion part-side protruding portion and the operation part-side protruding portion have triangular cross-sections protruding from outer peripheral surfaces of the insertion part-side operation member and the operation part-side operation member, respectively.

With the endoscope according to the aspect of the present invention, it is possible to provide an endoscope in which an operator can easily return an insertion part to a neutral position by rotationally moving the insertion part without visually confirming the endoscope.

### BRIEF DESCRIPTION OF THE DRAWINGS

Fig. 1 is a perspective view of an endoscope.
Fig. 2 is a perspective view of an endoscope operation part.
Fig. 3 is a perspective view of a periphery of an insertion part-side operation member and an operation part-side operation member.
Fig. 4 is a side view of the endoscope.
Fig. 5 is a cross-sectional view taken along line V-V of Fig. 4.
Fig. 6 is an enlarged cross-sectional view of main portions of Fig. 5, in which a dimension of each portion is shown.
Fig. 7 is a side view of the periphery of the insertion part-side operation member and the operation part-side operation member.
Fig. 8 is a perspective view of the insertion part-side operation member and the operation part-side operation member before the insertion part-side operation member and the operation part-side operation member are connected to each other.
Fig. 9 is a perspective view of the periphery of the insertion part-side operation member and the operation part-side operation member, in which the positions of the insertion part-side protruding portion and the operation part-side protruding portion are aligned with each other.
Fig. 10 is a view illustrating an example of an operation in a case where the insertion part-side operation member and the insertion part are returned to a neutral position (B) from a position (A) where the insertion part-side operation member and the insertion part are rotationally moved clockwise in a range of 0° to 90°.
Fig. 11 is a view illustrating an example of an operation to confirm that the insertion part-side protruding portion and the operation part-side protruding portion are in the neutral position by feel of a finger.
Fig. 12 is a view illustrating an example of an operation in a case where the insertion part-side operation member and the insertion part are returned to the neutral position (B) from a position (A) where the insertion part-side operation member and the insertion part are rotationally moved counterclockwise in a range of 0° to 90°.
Fig. 13 is a view illustrating an example of an operation in a case where the insertion part-side operation member and the insertion part are returned to the neutral position (B) from a position (A) where the insertion part-side operation member and the insertion part are rotationally moved clockwise by 90° or more.
Fig. 14 is a view illustrating an example of another operation in a case where the insertion part-side operation member and the insertion part are returned to the neutral position (B) from the position (A) where the insertion part-side operation member and the insertion part are rotationally moved clockwise in a range of 0° to 90°.

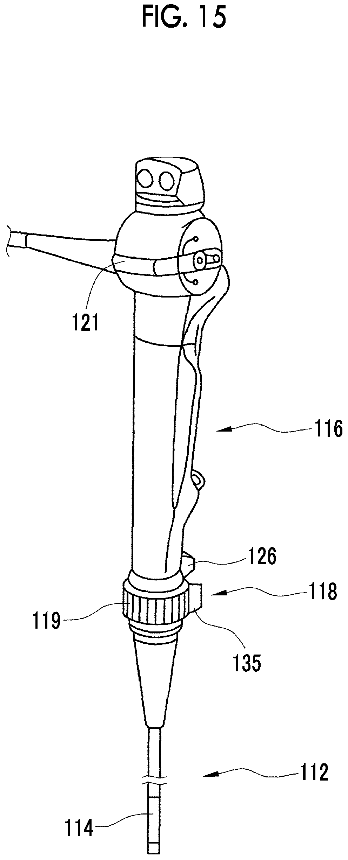

Fig. 15 is a perspective view of a conventional endoscope, which is provided with indicators indicating a rotational position of an insertion part.

### DESCRIPTION OF THE PREFERRED EMBODIMENTS

### [Outline Configuration of Endoscope]

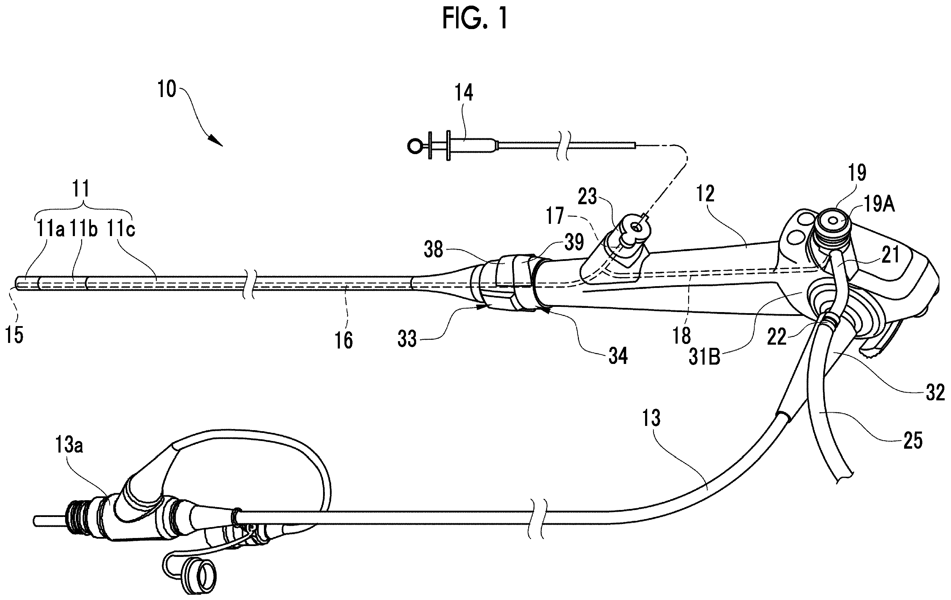

As shown in Fig. 1, an endoscope 10 is, for example, a bronchoscope that is inserted into the trachea, and comprises an insertion part 11 that is inserted into the trachea (corresponding to the inside of the subject in the claims), an operation part 12 consecutively provided at a proximal end portion of the insertion part 11, and a universal cord 13 connected to the operation part 12. The universal cord 13 is connected to an external device, such as a processor device or a light source device (not shown), via a complex type connector 13a.

The insertion part 11 consists of a distal end rigid portion 11a, a bendable portion 11b, and a flexible tube portion 11c having flexibility in this order from a distal end side to a proximal end side. An observation window or an illumination window (not shown) is provided on a distal end surface of the distal end rigid portion 11a, in addition to a forceps outlet 15 which is an outlet of a treatment tool 14, such as forceps. An image sensor (not shown) or the like is disposed on the inside of the observation window, and an optical fiber cable (not shown) is disposed on the inside of the illumination window. A signal line of the image sensor and the optical fiber cable are connected to the processor device and the light source device through the insertion part 11, the operation part 12, the universal cord 13, and the connector 13a, respectively. The bendable portion 11b is consecutively provided at the distal end rigid portion 11a, is located at the distal end portion when viewed from the entire insertion part 11, and is provided so as to be bendable.

A forceps channel 16 through which the treatment tool 14 is inserted is provided inside the insertion part 11. One end of the forceps channel 16 is connected to the forceps outlet 15, and the other end thereof is connected to a forceps port 17 provided in the operation part 12. The forceps channel 16 is also used as a route for sucking, for example, a body fluid, such as blood, and a solid matter, such as body wastes, from the forceps outlet 15. A suction channel 18 that branches from the forceps channel 16 is provided inside the operation part 12, and the suction channel 18 is connected to a suction button 19 provided in the operation part 12.

The suction button 19 comprises a pipe portion 21, a tube connection port 22, and a suction valve (not shown) that is provided therein. The suction valve is connected to the suction channel 18 in the operation part 12, and is connected to an external suction pump (not shown) of the operation part 12 via the pipe portion 21, the tube connection port 22, and a suction tube 25.

The shaft portion of the suction valve slides by the press operation of a press portion 19A of the suction button 19 so that the suction channel 18 and the pipe line of the suction pump communicate with each other. With this, a body fluid or the like can be sucked from the forceps outlet 15 of the insertion part 11 inserted into the subject or the like. In addition, the communication between the suction channel 18 and the pipe line of the suction pump is blocked by the release of the press operation of the press portion 19A so that the suction from the forceps outlet 15 can be stopped.

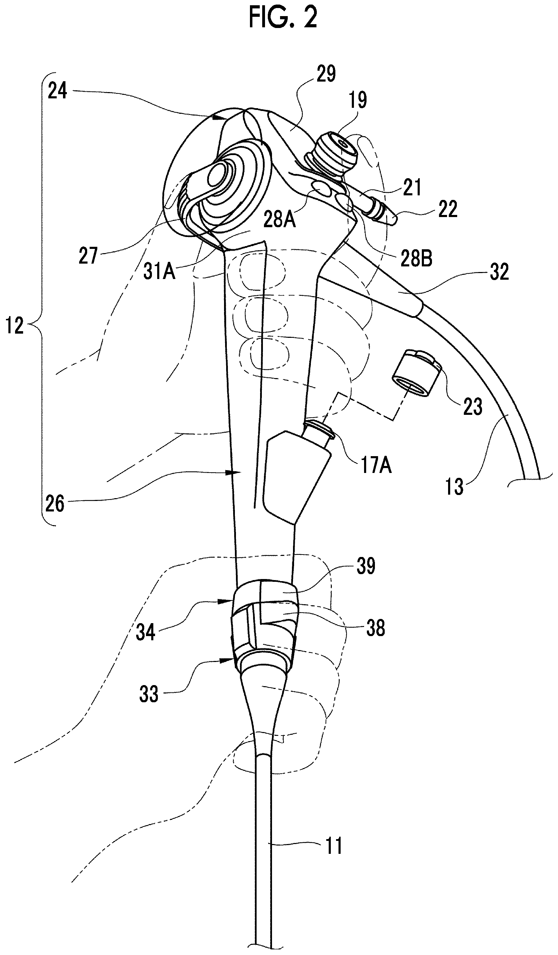

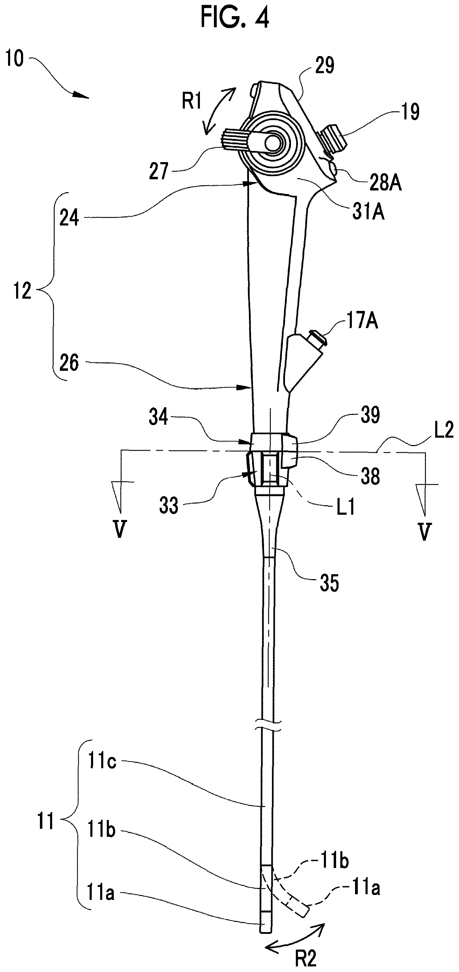

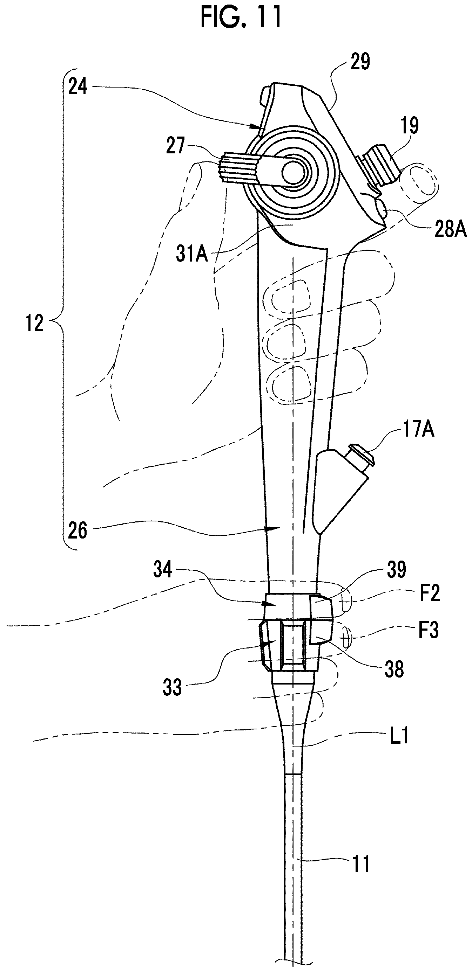

As shown in Fig. 2, the forceps port 17 is provided with a ferrule 17A, and a disposable type forceps valve 23 through which the treatment tool 14 can be inserted is mounted on the ferrule 17A. The forceps valve 23 prevents body fluids, wastes, air, and the like in the body from flowing back into the forceps channel 16 and leaking to the outside from the ferrule 17A. In Figs. 4 and 11, the forceps valve 23 is not shown.

### [Outline Configuration of Operation Part]

An operation part main body 24 and a grip portion 26 are integrally provided in the operation part 12. Operation members, such as a bending operation lever 27, push button switches 28A and 28B, and the suction button 19, are supported on the operation part main body 24. The bending operation lever 27 is an operation member that is used to bend the bendable portion 11b. The push button switches 28Aand 28B and the like are assigned any of functions, such as freeze, trigger, record, photometry mode switching, shutter speed switching, special light simple switching, and structure enhancement.

The operation part main body 24 is formed in a substantially box shape, and has a front surface 29 and side surfaces 31A and 31B continuous with the front surface 29. The suction button 19 is attached to the front surface 29. Further, the push button switches 28A and 28B are disposed on the front surface 29.

One side surface 31A is provided with the bending operation lever 27. The bending operation lever 27 is formed in an L shape that bends from the one side surface 31A toward the rear surface side of the operation part main body 24. A drive mechanism provided inside the operation part 12 operates by the rotational movement operation of the bending operation lever 27 so that the bendable portion 11b is operated to bend.

The universal cord 13 protrudes on the other side surface 31B (see Fig. 1), and a rubber sleeve 32 covering the mount portion of the universal cord 13 is disposed between the universal cord 13 and the operation part main body 24. The grip portion 26 has a substantially elongated tubular shape. Further, in the grip portion 26, the ferrule 17A is disposed near the insertion part 11.

### [Configurations of Insertion Part-Side Operation Member and Operation Part-Side Operation Member]

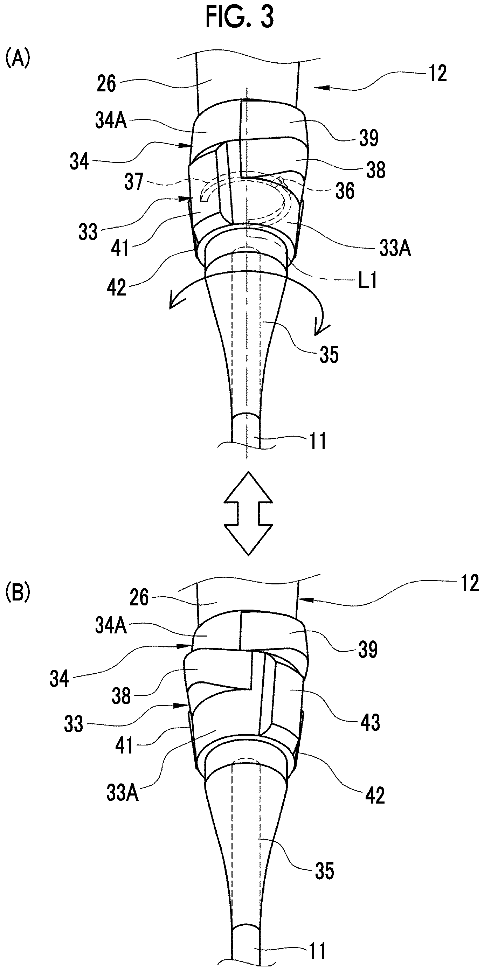

As shown in (A) and (B) of Fig. 3, an insertion part-side operation member 33 is provided at the proximal end portion of the insertion part 11, and an operation part-side operation member 34 is provided at the distal end portion of the operation part 12. The insertion part-side operation member 33 and the operation part-side operation member 34 function as indicators indicating the rotational movement position of the insertion part 11 with respect to the operation part 12, as will be described later.

The operation part-side operation member 34 is fixed to the distal end of the grip portion 26. The insertion part-side operation member 33 is fixed to the proximal end of the insertion part 11. A bending-proof portion 35 that is used to prevent the insertion part 11 from bending is externally mounted on the outer peripheral surface of the proximal end portion of the insertion part 11 at a position in contact with the insertion part-side operation member 33.

The insertion part-side operation member 33 is connected to the operation part-side operation member 34 so as to be rotationally movable. The insertion part-side operation member 33 is rotationally moved with respect to the operation part-side operation member 34 so that the insertion part 11 is rotationally movable around a rotational movement axis L1 with respect to the operation part 12. The rotational movement axis L1 coincides with the center line at the proximal end portion of the insertion part 11. That is, the rotational movement axis L1 is parallel to the insertion direction of the insertion part 11.

The insertion part-side operation member 33 and the operation part-side operation member 34 are formed in a substantially cylindrical shape. An outer peripheral surface 33A of the insertion part-side operation member 33 has a taper of which the outer diameter gradually decreases toward the distal end side of the insertion part 11. On the other hand, an outer peripheral surface 34A of the operation part-side operation member 34 has a taper of which the outer diameter gradually decreases toward the proximal end side of the operation part 12. The maximum outer diameter of the outer peripheral surface 33A is equal to the maximum outer diameter of the outer peripheral surface 34A.

A restriction pin 36 is provided inside the insertion part-side operation member 33. The restriction pin 36 is located inside a restriction groove 37 formed on a side of the operation part 12. The restriction groove 37 extends along the periphery of the rotational movement axis L1. The restriction pin 36 is movably loosely fitted inside the restriction groove 37. With this, the angle at which the insertion part-side operation member 33 is rotationally moved with respect to the operation part-side operation member 34 is restricted.

The insertion part-side operation member 33 has an insertion part-side protruding portion 38, and the operation part-side operation member 34 has an operation part-side protruding portion 39. The insertion part-side protruding portion 38 protrudes to the front surface side with respect to the operation part 12. The positions of the insertion part-side protruding portion 38 and the operation part-side protruding portion 39 are aligned with each other, so that the insertion part 11 can be set to the neutral position (a state shown in (A) of Fig. 3). Therefore, the insertion part-side protruding portion 38 and the operation part-side protruding portion 39 serve as indicators indicating the rotational movement position of the insertion part 11 with respect to the operation part 12. Further, the position of the insertion part-side protruding portion 38 shifts from the operation part-side protruding portion 39, so that the insertion part 11 is rotationally movable around the rotational movement axis L1 from the neutral position (a state shown in (B) of Fig. 3).

As shown in Fig. 4, the neutral position refers to a position in which a plane including a rotational movement direction R1 of the bending operation lever 27 and a plane including a bending direction R2 of the bendable portion 11b are the same as or parallel to each other in a case where the bending operation lever 27 is operated to move rotationally from a state in which the insertion part 11 has a linear shape and has no twist.

In a case where the insertion part 11 is inserted into the subject, the operator who operates the endoscope cannot visually confirm the bending state of the bendable portion 11b. However, in a case where the insertion part 11 is in the neutral position, the bending direction R2 of the bendable portion 11b can be estimated to some extent from the rotational movement direction R1 of the bending operation lever 27. Therefore, the operator who operates the endoscope often performs an operation to return the insertion part 11 to the neutral position.

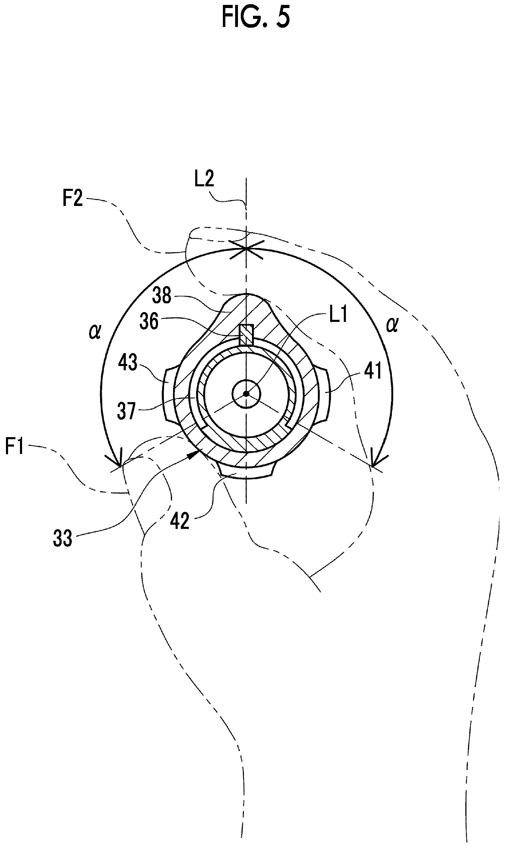

As shown in Fig. 5, the insertion part-side operation member 33 has three finger rest protruding portions 41 to 43 together with the insertion part-side protruding portion 38. The finger rest protruding portions 41 to 43 are rectangular protruding portions disposed in parallel to the rotational movement axis L1 when viewed from the radial direction of the insertion part-side operation member 33. The four sides forming the finger rest protruding portions 41 to 43 are each formed in a chamfered shape or a curved surface shape that is concave inward.

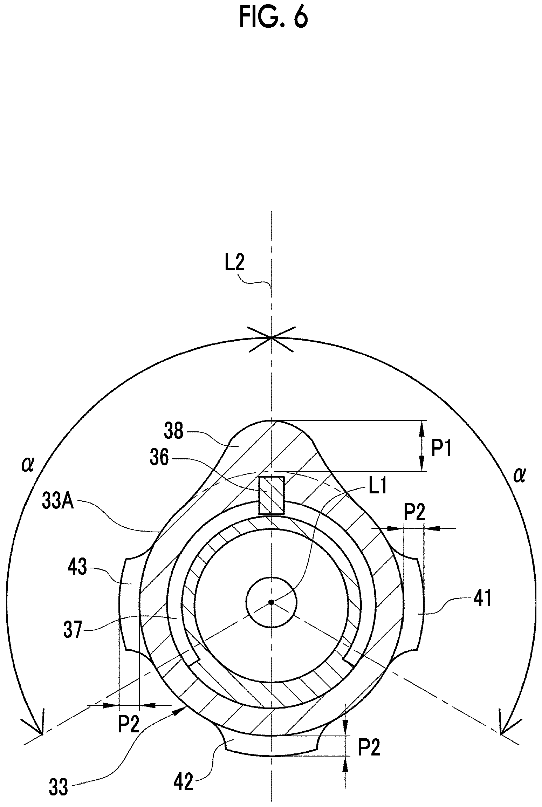

Among the finger rest protruding portions 41 to 43, the finger rest protruding portion 42 is provided at a position on a side opposite to the insertion part-side protruding portion 38 with the rotational movement axis L1 as the center, and the finger rest protruding portions 41 and 43 are located at positions 90° apart from the insertion part-side protruding portion 38 with the rotational movement axis L1 as the center and are provided at positions symmetrical to each other with respect to a center line L2 of the insertion part-side protruding portion 38. With this, the insertion part-side protruding portion 38 and the finger rest protruding portions 41 to 43 are disposed at X-shaped positions centered on the rotational movement axis L1. The center line L2 shown in Figs. 5 and 6 is a center line orthogonal to the rotational movement axis L1 and passing through the center of the operation part-side protruding portion 39 and the insertion part-side protruding portion 38 in a case where the insertion part 11 is in the neutral position.

For example, the operator puts the thumb F1 on the finger rest protruding portion 42, puts the index finger F2 on the insertion part-side protruding portion 38, and performs an operation to rotationally move the insertion part-side operation member 33. The operation when the operator performs an operation to rotationally move the insertion part-side operation member 33 will be described later. Further, in the example shown in the present embodiment, an example is shown in which the operator grips the operation part 12 with the left hand and performs an operation to rotationally move the insertion part-side operation member 33 with the right hand, in all cases, but the present invention is not limited thereto, and the operator may grip the operation part 12 with the right hand and may perform an operation to rotationally move the insertion part-side operation member 33 with the left hand.

As described above, although the insertion part-side operation member 33 is restricted in the angle of rotational movement with respect to the operation part-side operation member 34 by the restriction pin 36 and the restriction groove 37 loosely fitted with the restriction pin 36, specifically, the insertion part-side operation member 33 is rotationally movable clockwise and counterclockwise from the neutral position within respective predetermined angle ranges α. In the examples shown in Figs. 5 and 6, the angle range α is 120°.

As shown in Fig. 6, a second protrusion amount P2 in which the finger rest protruding portion 41 protrudes from the outer peripheral surface 33A of the insertion part-side operation member 33 in the radial direction passing through the finger rest protruding portion 41 is smaller than a first protrusion amount P1 in which the insertion part-side protruding portion 38 protrudes from the outer peripheral surface 33A of the insertion part-side operation member 33 in the radial direction passing through the insertion part-side protruding portion 38. The finger rest protruding portions 42 and 43 also have each the same second protrusion amount P2 as the finger rest protruding portion 41. With this, in a case where the operator touches the insertion part-side protruding portion 38 and the finger rest protruding portions 41 to 43 with a finger, it is possible to prevent the operator from mistaking the finger rest protruding portions 41 to 43 and the insertion part-side protruding portion 38 for each other.

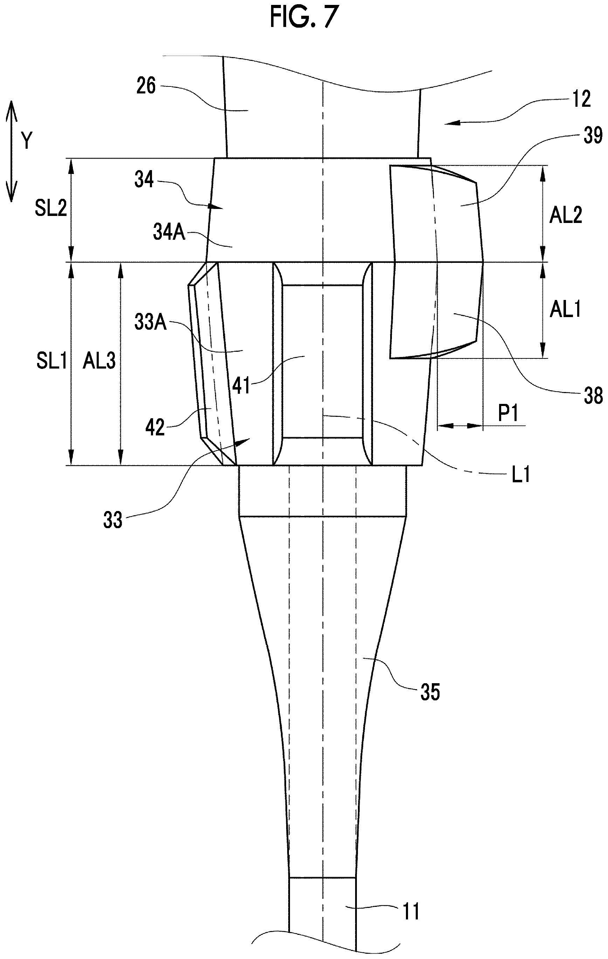

An alternating long-dash and short-dash line in Fig. 6 is an extension line of the outer peripheral surface 33A. As described above, since the outer peripheral surface 33Ahas a taper, the first protrusion amount P1 and the second protrusion amount P2 are the amounts of protrusion from the maximum outer diameter of the outer peripheral surface 33A. Further, the protrusion amount in which the operation part-side protruding portion 39 protrudes from the outer peripheral surface 34A of the operation part-side operation member 34 in the radial direction passing through the operation part-side protruding portion 39 is also the same as that of the first protrusion amount P1 (see Fig. 7).

As shown in Fig. 7, an axial length SL1 of the insertion part-side operation member 33 is longer than an axial length SL2 of the operation part-side operation member 34, in a Y-axis direction along the rotational movement axis L1. In the Y-axis direction, a length AL1 of the insertion part-side protruding portion 38 and the length AL2 of the operation part-side protruding portion 39 are equal to each other, and a length AL3 of each of the finger rest protruding portions 41 to 43 is greater than the length AL1 of the insertion part-side protruding portion 38. With this, it is possible to further prevent the operator from mistaking the finger rest protruding portions 41 to 43 and the insertion part-side protruding portion 38 for each other. In the present embodiment, the length AL3 of each of the finger rest protruding portions 41 to 43 is equal to the axial length SL1 of the insertion part-side operation member 33, in the Y-axis direction.

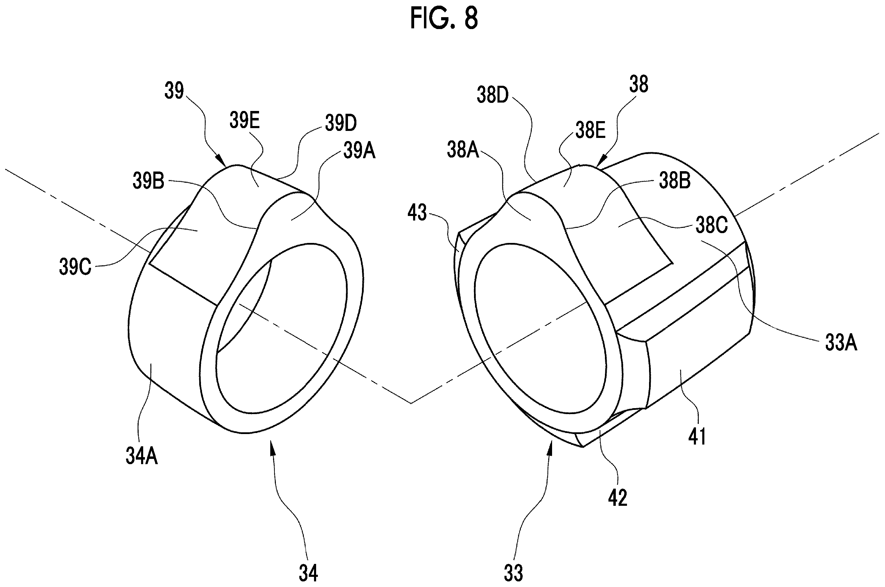

As shown in Fig. 8, in a case where the insertion part-side operation member 33 and the operation part-side operation member 34 are connected to each other, the insertion part-side protruding portion 38 and the operation part-side protruding portion 39 have end surfaces 38A and 39A facing each other, respectively. The insertion part-side protruding portion 38 and the operation part-side protruding portion 39 have triangular cross-sections protruding from the outer peripheral surfaces 33A and 34A, respectively. That is, the end surfaces 38A and 39A also have triangle shapes protruding from the outer peripheral surfaces 33A and 34A, respectively, and have the same shape. In Fig. 8, in order to prevent the drawing from being complicated, the insertion part 11, the operation part 12, and the like are not shown.

As described above, the maximum outer diameters of the outer peripheral surfaces 33A and 34A are the same, and the insertion part-side protruding portion 38 and the operation part-side protruding portion 39 also have the same first protrusion amount P1, and also have the same triangular cross-sections protruding from the outer peripheral surfaces 33A and 34A, respectively. That is, outer contour lines 38B and 39B of the end surfaces 38A and 39A of the insertion part-side protruding portion 38 and the operation part-side protruding portion 39 coincide with each other in a case where the end surfaces 38A and 39A face each other.

The insertion part-side protruding portion 38 has side surfaces 38C and 38D and a distal end peripheral surface 38E of which the triangular distal end portion is formed in a semicircular shape. The side surfaces 38C and 38D are surfaces continuous with the outer peripheral surface 33A. The distal end peripheral surface 38E is located between the side surfaces 38C and 38D, and is smoothly continuous with the side surfaces 38C and 38D. The side surfaces 38C and 38D and the distal end peripheral surface 38E are connected to the end surface 38A via the contour line 38B.

The operation part-side protruding portion 39 has side surfaces 39C and 39D and a distal end peripheral surface 39E of which the triangular distal end portion is formed in a semicircular shape, as in the insertion part-side protruding portion 38. The side surfaces 39C and 39D are surfaces continuous with the outer peripheral surface 34A. The side surfaces 39C and 39D and the distal end peripheral surface 39E are connected to the end surface 39A via the contour line 39B.

The side surfaces 38C and 38D of the insertion part-side protruding portion 38 are curved surfaces that are concave toward the center line of the insertion part-side protruding portion 38. Meanwhile, the side surfaces 39C and 39D of the operation part-side protruding portion 39 are also curved surfaces that are concave toward the center line of the operation part-side protruding portion 39. With this, the operator easily puts the fingertip portion on the insertion part-side protruding portion 38 and the operation part-side protruding portion 39.

The contour line 38B is not actually a line but has a small rounded shape that smoothly connects the end surface 38A to the side surfaces 38C and 38D and the distal end peripheral surface 38E. Similarly, the contour line 39B also has a small rounded shape that smoothly connects the end surface 39A to the side surfaces 39C and 39D and the distal end peripheral surface 39E.

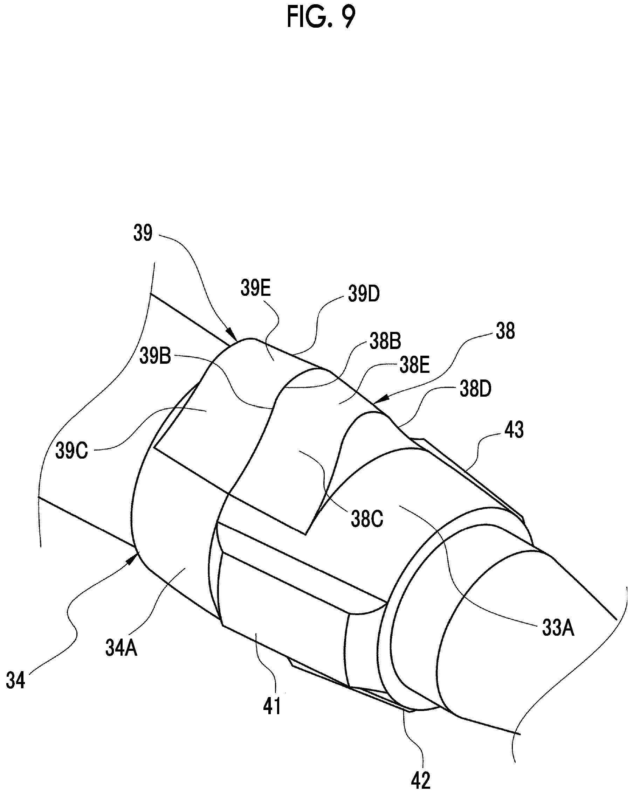

As shown in Fig. 9, the outer peripheral surface (including the side surfaces 38C and 38D and the distal end peripheral surface 38E) constituting the insertion part-side protruding portion 38 and the outer peripheral surface (including the side surfaces 39C and 39D and the distal end peripheral surface 39E) constituting the operation part-side protruding portion 39 constitute a continuous curved surface, in a case where the end surfaces 38A and 39A of the insertion part-side protruding portion 38 and the operation part-side protruding portion 39 face each other, and the contour lines 38B and 39B coincide with each other. Then, in a case where the end surfaces 38A and 39A face each other and the contour lines 38B and 39B coincide with each other in this way, the insertion part 11 is located at the above-mentioned neutral position.

The operation in a case where the operator returns the insertion part-side operation member 33 and the insertion part 11, which are rotationally moved from the neutral position, to the neutral position when using the endoscope will be described. For example, in a case where the operator who operates the endoscope inserts the endoscope into the subject and ends the observation, or in a case where the operator performs an operation to remove the insertion part 11 from the subject, the operator returns the insertion part-side operation member 33 and the insertion part 11 to the neutral position once, in many cases.

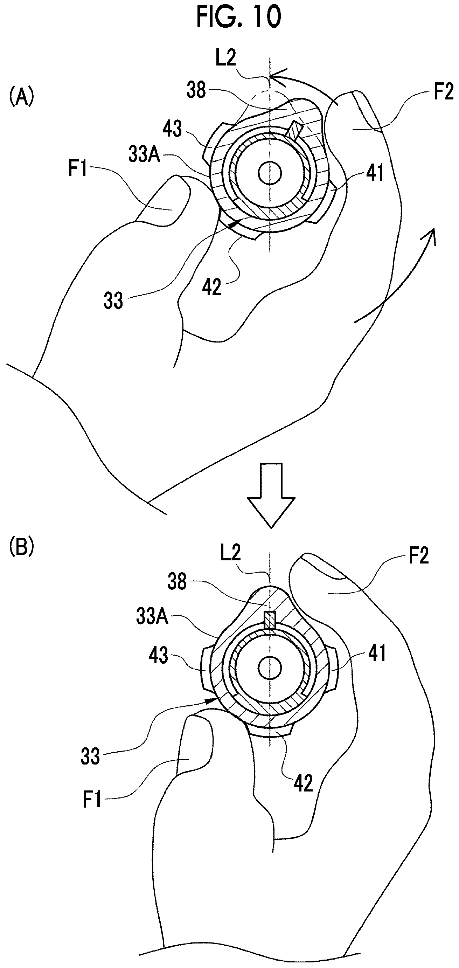

In the example shown in (A) of Fig. 10, the operator tries to return the insertion part-side operation member 33 and the insertion part 11 to the neutral position from the position where the insertion part-side operation member 33 and the insertion part 11 are rotationally moved clockwise in the range of 0° to 90°. In this case, the operator puts the thumb F1 on the finger rest protruding portion 42, puts the index finger F2 on the insertion part-side protruding portion 38, and performs an operation to rotationally move the insertion part-side operation member 33 counterclockwise. At this time, since the finger rest protruding portion 42 is near the rear surface side of the operation part 12 and the insertion part-side protruding portion 38 is near the front surface side of the operation part 12, the finger rest protruding portion 42 and the insertion part-side protruding portion 38 are located at positions where the operator easily puts the thumb F1 and the index finger F2 thereon, respectively.

In a case where the rotational movement operation is performed in this way, the finger rest protruding portion 42 is provided at a position on a side opposite to the insertion part-side protruding portion 38, so that the operator can easily put the finger thereon and can easily return the insertion part-side operation member 33 and the insertion part 11 to the neutral position by rotationally moving the insertion part-side operation member 33 and the insertion part 11 (a state shown in (B) of Fig. 10). Then, as described above, the positions of the insertion part-side protruding portion 38 and the operation part-side protruding portion 39 are aligned with each other. Since the insertion part-side protruding portion 38 and the operation part-side protruding portion 39 themselves serve as indicators indicating the rotational movement position of the insertion part 11 with respect to the operation part 12, the insertion part 11 can be easily returned to the neutral position. In this case, as shown in Fig. 11, it is possible to confirm that the insertion part-side protruding portion 38 and the operation part-side protruding portion 39 are in the neutral position only by the feel of a finger, for example, by touching the positions of the insertion part-side protruding portion 38 and the operation part-side protruding portion 39 with the pads of the index finger F2 and the middle finger F3 of the operator. With this, the operator does not need to visually confirm the endoscope 10.

Further, as described above, since the contour lines 38B and 39B of the end surfaces 38A and 39A of the insertion part-side protruding portion 38 and the operation part-side protruding portion 39, of which the positions are set to the neutral position, coincide with each other in a case where the end surfaces 38A and 39A face each other, it is easy to confirm that the insertion part-side protruding portion 38 and the operation part-side protruding portion 39 are in the neutral position only by the feel of a finger. Further, in a case where the contour lines 38B and 39B coincide with each other, the outer peripheral surface constituting the insertion part-side protruding portion 38 and the outer peripheral surface constituting the operation part-side protruding portion 39 constitute a continuous curved surface. Therefore, there is no difference in level between the insertion part-side protruding portion 38 and the operation part-side protruding portion 39, and it is easier to confirm that the insertion part-side protruding portion 38 and the operation part-side protruding portion 39 are in the neutral position only by the feel of a finger.

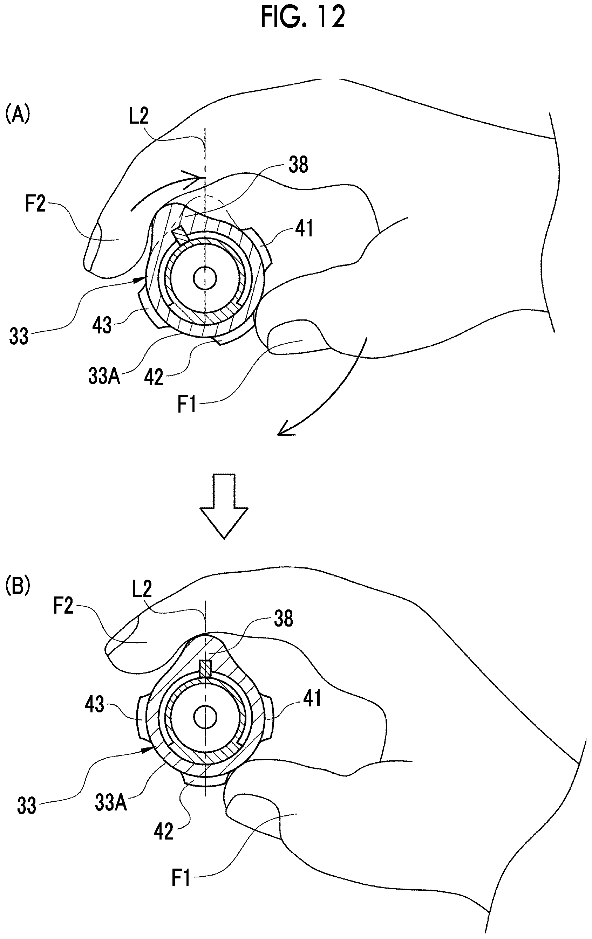

Further, in the example shown in (A) of Fig. 12, the operator tries to return the insertion part-side operation member 33 and the insertion part 11 to the neutral position from the position where the insertion part-side operation member 33 and the insertion part 11 are rotationally moved counterclockwise in the range of 0° to 90°. In this case as well, the operator puts the thumb F1 on the finger rest protruding portion 42, puts the index finger F2 on the insertion part-side protruding portion 38, and performs an operation to rotationally move the insertion part-side operation member 33 clockwise.

As in the examples shown in (A) and (B) of Fig. 10, the finger rest protruding portion 42 is provided at a position on a side opposite to the insertion part-side protruding portion 38, so that the operator can easily put the finger thereon and can easily return the insertion part-side operation member 33 and the insertion part 11 to the neutral position by rotationally moving the insertion part-side operation member 33 and the insertion part 11 (a state shown in (B) of Fig. 12). Then, since it is possible to confirm that the insertion part-side operation member 33 and the insertion part 11 are in the neutral position only by the feel of a finger, the operator does not need to visually confirm the endoscope 10.

Meanwhile, in a case where the operator returns the insertion part-side operation member 33 and the insertion part 11 to the neutral position from the position where the insertion part-side operation member 33 and the insertion part 11 are rotationally moved clockwise or counterclockwise by 90° or more, it is better to use the finger rest protruding portion 41 or the finger rest protruding portion 43 to perform the rotational movement operation.

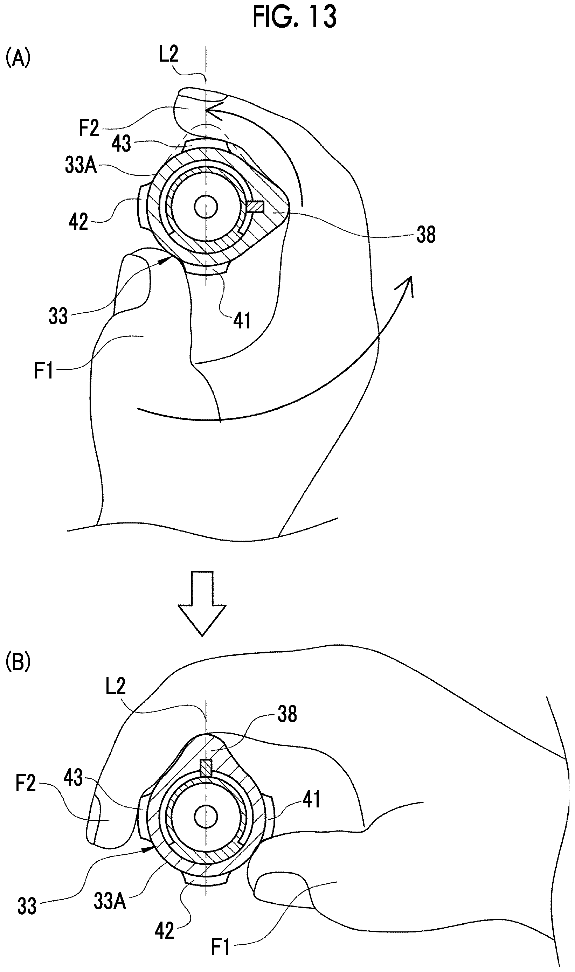

In the example shown in (A) of Fig. 13, the operator tries to return the insertion part-side operation member 33 and the insertion part 11 to the neutral position from the position where the insertion part-side operation member 33 and the insertion part 11 are rotationally moved clockwise by 90° or more. In this case, for example, the operator puts the thumb F1 on the finger rest protruding portion 41, puts the middle (near the first joint or the second joint) of the index finger F2 on the insertion part-side protruding portion 38, and performs an operation to rotationally move the insertion part-side operation member 33 counterclockwise. At this time, since the finger rest protruding portion 41 is near the rear surface side of the operation part 12 and the insertion part-side protruding portion 38 is near the side surface side of the operation part 12, the finger rest protruding portion 41 and the insertion part-side protruding portion 38 are located at positions where the operator easily puts the middle of the thumb F1 and the index finger F2 thereon, respectively. Further, in this case, the operator may put the pad of the index finger F2 on the finger rest protruding portion 43 provided at a position symmetrical to the finger rest protruding portion 41.

In a case where the rotational movement operation is performed in this way, the finger rest protruding portion 41 and the finger rest protruding portion 43 are provided at positions 90° apart from the insertion part-side protruding portion 38 with the rotational movement axis L1 as the center, so that the operator can easily put the finger thereon and can easily return the insertion part-side operation member 33 and the insertion part 11 to the neutral position by rotationally moving the insertion part-side operation member 33 and the insertion part 11 (a state shown in (B) of Fig. 13). Then, it is possible to confirm that the insertion part-side operation member 33 and the insertion part 11 are in the neutral position only by the feel of a finger.

In a case where the insertion part-side operation member 33 and the insertion part 11 are returned to the neutral position from the position where the insertion part-side operation member 33 and the insertion part 11 are rotationally moved counterclockwise by 90° or more, the operator need only put the thumb F1 on the finger rest protruding portion 43, put the index finger F2 on the insertion part-side protruding portion 38, and perform an operation to rotationally move the insertion part-side operation member 33 clockwise. With this, it is possible to easily return the insertion part-side operation member 33 and the insertion part 11 to the neutral position by rotationally moving the insertion part-side operation member 33 and the insertion part 11, as in the examples shown in (A) and (B) of Fig. 13. Then, it is possible to confirm that the insertion part-side operation member 33 and the insertion part 11 are in the neutral position only by the feel of a finger.

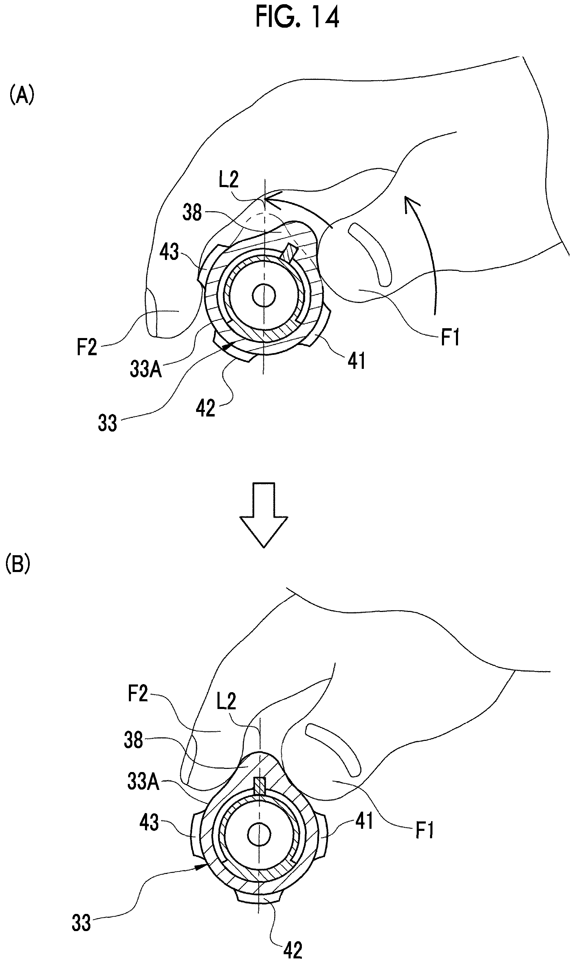

The operation method for returning the insertion part-side operation member 33 and the insertion part 11 to the neutral position is not limited to the operation method exemplified in the above embodiment. In the example shown in (A) of Fig. 14, the operator puts the pad of the thumb F1 on the insertion part-side protruding portion 38, puts the index finger F2 on the finger rest protruding portion 43, and performs an operation to rotationally move the insertion part-side operation member 33 counterclockwise. In this example, the operator tries to return the insertion part-side operation member 33 and the insertion part 11 to the neutral position from the position where the insertion part-side operation member 33 and the insertion part 11 are rotationally moved clockwise in the range of 0° to 90°. Further, as in the operation method of the above embodiment, the operator grips the operation part 12 with the left hand, and performs an operation to rotationally move the insertion part-side operation member 33 with the right hand.

In a case where the rotational movement operation is performed in this way, the finger rest protruding portion 43 is provided at position 90° apart from the insertion part-side protruding portion 38 with the rotational movement axis L1 as the center, so that the operator can also easily put the finger thereon and can also easily return the insertion part-side operation member 33 and the insertion part 11 to the neutral position by rotationally moving the insertion part-side operation member 33 and the insertion part 11 (a state shown in (B) of Fig. 14). Then, the operator aligns the positions of the insertion part-side protruding portion 38 and the operation part-side protruding portion 39 with each other. Further, in this case, it is possible to confirm that the insertion part-side protruding portion 38 and the operation part-side protruding portion 39 are in the neutral position by the touch with the pad of the thumb F1 or by the pinch with the pad of the thumb F1 and the pad of the index finger F2. With this, the operator does not need to visually confirm the endoscope 10.

In the above embodiment, three finger rest protruding portions are provided in the insertion part-side operation member, but the present invention is not limited thereto, and at least one or more need only be provided. Further, in a case where there is one finger rest protruding portion, it is preferable to provide at least the finger rest protruding portion 42 that is located at a position on a side opposite to the insertion part-side protruding portion 38 with the rotational movement axis L1 as the center. Further, in the above embodiment, the finger rest protruding portions 41 and 43 are provided at positions 90° apart from the insertion part-side protruding portion 38 with the rotational movement axis L1 as the center, but at least one need only be provided.

In the above embodiment, two finger rest protruding portions, among the finger rest protruding portions, are provided at positions 90° apart from the insertion part-side protruding portion 38 with the rotational movement axis L1 as the center, and are provided at positions symmetrical to each other with respect to the center line L2, but the present invention is not limited thereto, and for example, the two finger rest protruding portions may be provided at positions 120° apart from the insertion part-side protruding portion 38 and at positions symmetrical to each other with respect to the center line L2.

In the above embodiment, the endoscope 10 is inserted into the trachea has been described as an example, but the present invention can also be applied to an endoscope used for, for example, various medical endoscopes, such as an otolaryngology endoscope and a bladder endoscope, and other uses, such as industrial applications.

### Explanation of References

10: endoscope
11: insertion part
11a: distal end rigid portion
11b: bendable portion
11c: flexible tube portion
12: operation part
13: universal cord
13a: connector
14: treatment tool
15: forceps outlet
16: forceps channel
17: forceps port
17A: ferrule
18: suction channel
19: suction button
19A: press portion
21: pipe portion
22: tube connection port
23: forceps valve
24: operation part main body
25: suction tube
26: grip portion
27: bending operation lever
28A: push button switch
28B: push button switch
29: front surface
31A: side surface
31B: side surface
32: sleeve
33: insertion part-side operation member
33A: outer peripheral surface
34: operation part-side operation member
34A: outer peripheral surface
35: bending-proof portion
36: restriction pin
37: restriction groove
38: insertion part-side protruding portion
38A: end surface
38B: contour line
38C, 38D: side surface
38E: distal end peripheral surface
39: operation part-side protruding portion
39A: end surface
39B: contour line
39C, 39D: side surface
39E: distal end peripheral surface
41, 42, 43: finger rest protruding portion
112: insertion part
114: bendable portion
116: operation part
118: connection portion
119: rotary knob
121: bending lever
126: cover protruding portion
135: protruding portion
AL1, AL2, AL3: length
F1: thumb
F2: index finger
F3: middle finger
L1: rotational movement axis
L2: center line
P1: first protrusion amount
P2: second protrusion amount
R1: rotational movement direction
R2: bending direction
SL1, SL2: axial length
α: angle range

## Claims

1. An endoscope comprising:
an insertion part (11) that is inserted into a subject;
an operation part (12) consecutively provided at a proximal end portion of the insertion part;
a bendable portion (11b) provided at a distal end portion of the insertion part so as to be bendable;
an insertion part-side operation member (33) provided at the proximal end portion of the insertion part; and
an operation part-side operation member (34) provided at a distal end portion of the operation part,
wherein the insertion part-side operation member (33) is connected to the operation part-side operation member (34) so as to be rotationally movable,
the insertion part-side operation member (33) is rotationally moved with respect to the operation part-side operation member (34) so that the insertion part is rotationally movable around a rotational movement axis parallel to an insertion direction with respect to the operation part,
the operation part-side operation member (34) has an operation part-side protruding portion (39),
the insertion part-side operation member (33) has an insertion part-side protruding portion (38) and a finger rest protruding portion (41, 42, 43) provided at least at a position on a side opposite to the insertion part-side protruding portion with the rotational movement axis as a center, and
the insertion part-side protruding portion (33) and the operation part-side protruding portion (39) serve as indicators indicating a rotational movement position of the insertion part with respect to the operation part, and the endoscope is **characterised in that**:
a length of the finger rest protruding portion (41, 42, 43) is greater than a length of the insertion part-side protruding portion (38), in an axial direction along the rotational movement axis.

2. The endoscope according to claim 1,
wherein the operation part (12) is provided with a bending operation lever that is operated to move rotationally, and
the insertion part (11) is located at a neutral position in which a plane including a rotational movement direction of the bending operation lever and a plane including a bending direction of the bendable portion are the same as or parallel to each other, in a case where the end surfaces of the operation part-side protruding portion and the insertion part-side protruding portion face each other and the contour lines coincide with each other.

3. The endoscope according to any one of claims 1 or 2,
wherein a plurality of the finger rest protruding portions (41, 42, 43) are provided on the insertion part-side operation member, and are provided at positions symmetrical to each other with respect to a center line of the insertion part-side protruding portion orthogonal to the rotational movement axis.

4. The endoscope according to any one of claims1 to 3,
wherein three finger rest protruding portions (41, 42, 43) are provided on the insertion part-side operation member, and
the insertion part-side protruding portion and the finger rest protruding portions are disposed in an X shape centered on the rotational movement axis.

5. The endoscope according to any one of claims 1 to 4,
wherein the insertion part-side protruding portion (38) and the operation part-side protruding portion (39) have triangular cross-sections protruding from outer peripheral surfaces of the insertion part-side operation member and the operation part-side operation member, respectively.

## Patentansprüche

1. Endoskop, umfassend:
einen Einführteil (11), der in eine Untersuchungsperson eingeführt wird;
einen Bedienteil (12), der nacheinander an einem proximalen Endabschnitt des Einführteils vorgesehen ist;
einen biegsamen Abschnitt (11b), der an einem distalen Endabschnitt des Einführteils so vorgesehen ist, dass er biegsam ist;
ein einführteilseitiges Bedienelement (33), das an dem proximalen Endabschnitt des Einführteils vorgesehen ist; und
ein bedienteilseitiges Bedienelement (34), das an einem distalen Endabschnitt des Bedienteils vorgesehen ist,
wobei das einführteilseitige Bedienelement (33) mit dem bedienteilseitigen Bedienelement (34) so verbunden ist, dass es drehbeweglich ist,
das einführteilseitige Bedienelement (33) in Bezug auf das bedienteilseitige Bedienelement (34) so drehend bewegt wird, dass der Einführteil in Bezug auf den Bedienteil um eine Drehbewegungsachse parallel zu einer Einführrichtung drehbeweglich ist,
das bedienteilseitige Bedienelement (34) einen bedienteilseitigen vorstehenden Abschnitt (39) aufweist,
das einführteilseitige Bedienelement (33) einen einführteilseitigen vorstehenden Abschnitt (38) und einen vorstehenden Fingerauflageabschnitt (41, 42, 43), der mindestens an einer Position auf einer Seite, die dem einführteilseitigen vorstehenden Abschnitt gegenüberliegt, mit der Drehbewegungsachse als einer Mitte vorgesehen ist, aufweist, und
der einführteilseitige vorstehende Abschnitt (33) und der bedienteilseitige vorstehende Abschnitt (39) als Indikatoren dienen, die eine Drehbewegungsposition des Einführteils in Bezug auf den Bedienteil angeben, und das Endoskop **dadurch gekennzeichnet ist, dass**:
eine Länge des vorstehenden Fingerauflageabschnitts (41, 42, 43) in einer Axialrichtung entlang der Drehbewegungsachse größer als eine Länge des einführteilseitigen vorstehenden Abschnitts (38) ist.

2. Endoskop nach Anspruch 1,
wobei der Bedienteil (12) mit einem Biegebetätigungshebel versehen ist, der betätigt wird, um sich drehend zu bewegen, und
der Einführteil (11) sich in einem Fall, in dem die Endflächen des bedienteilseitigen vorstehenden Abschnitts und des einführteilseitigen vorstehenden Abschnitts einander zugewandt sind und die Konturlinien miteinander übereinstimmen, an einer neutralen Position befindet, in der eine Ebene, die eine Drehbewegungsrichtung des Biegebetätigungshebels enthält, und eine Ebene, die eine Biegerichtung des biegsamen Abschnitts enthält, gleich oder parallel zueinander sind.

3. Endoskop nach einem der Ansprüche 1 oder 2,
wobei mehrere der vorstehenden Fingerauflageabschnitte (41, 42, 43) an dem einführteilseitigen Bedienelement vorgesehen sind und an Positionen vorgesehen sind, die symmetrisch zueinander in Bezug auf eine Mittellinie des einführteilseitigen vorstehenden Abschnitts senkrecht zu der Drehbewegungsachse sind.

4. Endoskop nach einem der Ansprüche 1 bis 3,
wobei drei vorstehende Fingerauflageabschnitte (41, 42, 43) an dem einführteilseitigen Bedienelement vorgesehen sind, und
der einführteilseitige vorstehende Abschnitt und die vorstehenden Fingerauflageabschnitte in einer X-Form, die auf der Drehbewegungsachse zentriert ist, angeordnet sind.

5. Endoskop nach einem der Ansprüche 1 bis 4,
wobei der einführteilseitige vorstehende Abschnitt (38) und der bedienteilseitige vorstehende Abschnitt (39) dreieckige Querschnitte, die entsprechend von Außenumfangsflächen des einführteilseitigen Bedienelements und des bedienteilseitigen Bedienelements vorstehen, aufweisen.

## Revendications

1. Endoscope comprenant :
une partie d'insertion (11) qui est insérée dans un sujet ;
une partie d'opération (12) consécutivement prévue à une partie d'extrémité proximale de la partie d'insertion ;
une partie flexible (11b) prévue à une partie d'extrémité distale de la partie d'insertion de manière à être flexible ;
un élément d'opération côté partie d'insertion (33) prévu à la partie d'extrémité proximale de la partie d'insertion ; et
un élément d'opération côté partie d'opération (34) prévu à une partie d'extrémité distale de la partie d'opération,
dans lequel l'élément d'opération côté partie d'insertion (33) est connecté à l'élément d'opération côté partie d'opération (34) de manière à être mobile par rotation,
l'élément d'opération côté partie d'insertion (33) est déplacé en rotation par rapport à l'élément d'opération côté partie d'opération (34) de sorte que la partie d'insertion soit mobile par rotation autour d'un axe de mouvement de rotation parallèle à une direction d'insertion par rapport à la partie d'opération,
l'élément d'opération côté partie d'opération (34) a une partie saillante côté partie d'opération (39),
l'élément d'opération côté partie d'insertion (33) a une partie saillante côté partie d'insertion (38) et une partie saillante de repos de doigt (41, 42, 43) prévue au moins à une position sur un côté opposé à la partie saillante côté partie d'insertion avec l'axe de mouvement de rotation comme centre, et
la partie saillante côté partie d'insertion (33) et la partie saillante côté partie d'opération (39) servent d'indicateurs indiquant une position de mouvement de rotation de la partie d'insertion par rapport à la partie d'opération, et l'endoscope est **caractérisé en ce que** :
une longueur de la partie saillante de repos de doigt (41, 42, 43) est supérieure à une longueur de la partie saillante côté partie d'insertion (38), dans une direction axiale le long de l'axe de mouvement de rotation.

2. Endoscope selon la revendication 1,
dans lequel la partie d'opération (12) est pourvue d'une manette d'opération de flexion qui est actionnée pour se déplacer par rotation, et
la partie d'insertion (11) est située à une position neutre dans laquelle un plan incluant une direction de mouvement de rotation de la manette d'opération de flexion et un plan incluant une direction de flexion de la partie flexible sont les mêmes ou parallèles l'un à l'autre, dans un cas où les surfaces d'extrémité de la partie saillante côté partie d'opération et la partie saillante côté partie d'insertion font face l'une à l'autre et les lignes de contour coïncident l'une avec l'autre.

3. Endoscope selon l'une quelconque des revendications 1 ou 2,
dans lequel une pluralité des parties saillantes de repos de doigt (41, 42, 43) sont prévues sur l'élément d'opération côté partie d'insertion, et sont prévues à des positions symétriques l'une par rapport à l'autre par rapport à une ligne centrale de la partie saillante côté partie d'insertion orthogonale à l'axe de mouvement de rotation.

4. Endoscope selon l'une quelconque des revendications 1 à 3,
dans lequel trois parties saillantes de repos de doigt (41, 42, 43) sont prévues sur l'élément d'opération côté partie d'insertion, et
la partie saillante côté partie d'insertion et les parties saillantes de repos de doigt sont disposées en forme de X centrée sur l'axe de mouvement de rotation.

5. Endoscope selon l'une quelconque des revendications 1 à 4,
dans lequel la partie saillante côté partie d'insertion (38) et la partie saillante côté partie d'opération (39) ont des sections transversales triangulaires saillant à partir de surfaces périphériques extérieures de l'élément d'opération côté partie d'insertion et de l'élément d'opération côté partie d'opération, respectivement.
